**Europäisches Patentamt**

**European Patent Office**

**Office européen des brevets**

(19)

(11) Veröffentlichungsnummer : **0 040 782**
**B1**

(12) # EUROPÄISCHE PATENTSCHRIFT

(45) Veröffentlichungstag der Patentschrift :
09.01.85

(51) Int. Cl.⁴ : **A 43 B 7/00**

(21) Anmeldenummer : **81103794.4**

(22) Anmeldetag : **18.05.81**

(54) **Orthopädisches Schuhwerk oder Schuhwerkteile.**

(30) Priorität : **22.05.80 DE 3019561**
**06.04.81 DE 3113820**

(43) Veröffentlichungstag der Anmeldung :
**02.12.81 Patentblatt 81/48**

(45) Bekanntmachung des Hinweises auf die Patenterteilung : **09.01.85 Patentblatt 85/02**

(84) Benannte Vertragsstaaten :
**AT BE CH DE FR GB IT LI LU NL SE**

(56) Entgegenhaltungen :
**DE-B- 1 261 778**
**DE-B- 2 842 815**
**FR-A- 2 203 264**
**US-A- 1 662 814**
**US-A- 2 390 416**

(73) Patentinhaber : **Dieterich, Alfred, Dr.**
**Westtorgraben 3**
**D-8500 Nürnberg (DE)**

(72) Erfinder : **Dieterich, Alfred, Dr.**
**Westtorgraben 3**
**D-8500 Nürnberg (DE)**

(74) Vertreter : **Richter, Bernhard, Dipl.-Ing.**
**Beethovenstrasse 10**
**D-8500 Nürnberg 20 (DE)**

EP 0 040 782 B1

Jouve, 18, rue St-Denis, 75001 Paris, France

**Beschreibung**

Die Erfindung betrifft ein orthopädisches Schuhwerk oder Schuhwerkteil mit einer Vorrichtung zur konservativen Behandlung von Mißbildungen des Fußes, wobei der Druck des Rückfußes auf das Schuhwerk oder Schuhwerkteil in eine intermittierende Kraftwirkung auf die zu behandelnden Abschnitte des Fußes umgewandelt wird. Eine orthopädische Sandale dieser Art (Oberbegriff des Anspruches 1) ist zur konservativen Behandlung von Hammerzehen und gegebenenfalls auch einer Korrektur der X-Stellung der Großzehe aus der DE-B-28 42 815 vorgesehen. Eine ähnliche orthopädische Sandale dieser Art ist Inhalt der älteren deutschen Patentanmeldung DE-A-30 16 425.

Die Aufgabe der Erfindung besteht primär darin, ein orthopädisches Schuhwerk oder Schuhwerkteile gemäß dem obengenannten Oberbegriff des Anspruches 1 so auszugestalten, daß eine ebenfalls intermittierende Korrekturbehandlung von solchen Mißbildungen des Fußes möglich ist, bei denen die Knochen des vorderen Fußabschnittes gegenüber dem Rückfuß (Ferse) im Sinne einer Zug- bzw. Druck- und/oder Drehbeanspruchung stellungsgemäß korrigiert werden. Ferner sollen die Mittel zur Durchführung dieser Korrekturen, einschließlich einer Korrektur des Hallux-Valgus (eine im Grundgelenk in X-Stellung teilversteifte Großzehe), die zumeist mit einer Erkrankung der Zehen zwei bis fünf in Form sogenannter Krallenzehen und Hammerzehen gekoppelt ist, so einfach wie möglich gestaltet werden, um nicht nur die Herstellungskosten zu senken, sondern auch die Anordnung für den praktischen Gebrauch möglichst einfach und robust zu machen.

Zur Lösung dieser Aufgabe sieht die Erfindung zunächst unter der Kraftwirkung stehende Mittel vor, die den Vorfußbereich um die Fußlängsachse drehen und/oder um eine senkrecht zur Sohle verlaufende Achse zur Seite bewegen, wobei die Mittel zur Verdrehung des Vorfußbereiches an der medialen Seite des Vorfußes im Sinne deren Absenkens angreifen. Dabei kann das Schuhwerk eine Sandale oder ein Schuh und das Schuhwerkteil eine in einen Schuh, in der Regel einen Sonderschuh, einzubringende Einlage sein, die nach der Erfindung ausgestaltet ist. Hiermit können und zwar entweder in der Sitzstellung, wobei der Fuß entsprechend rhythmisch gegen den Boden gedrückt wird oder aber, und dies ist die bevorzugte Therapie, beim Gehen durch den Gehvorgang selber folgende Fußdeformitäten konservativ und intermittierend behandelt werden :

Der Knick-Plattfuß läßt sich durch die Relativ-Bewegung in Form des Drehens oder Verwringens des Vorfußes gegenüber dem Rückfuß (Ferse) therapeutisch erfolgreich angreifen. Diese Deformität wird teils als Erbanlage, teils aber auch durch eine Fehlbeanspruchung bei einer bindegeweblichen Schwäche erworben. Beim Knickfuß steht die ansonsten senkrechte Achse der Ferse in Bezug zur senkrechten Achse des Unterschenkels in einer X-Stellung, d. h. beim Stehen mit geschlossenen Füßen bilden beide Beine eine « X »-Form.

Der sogenannte Plattfuß setzt sich aber zumeist aus dem Senkfuß, dem pes supinatus und vielfach auch noch dem Spreizfuß zusammen.

Unter dem Senkfuß versteht man die verminderte Wölbung des sogenannten Fuß-Längsgewölbes, die äußerlich durch ein Herabsinken der inneren (medialen) Fußseite erkennbar ist, unter dem Spreizfuß die verminderte Wölbung des sogenannten Quergewölbes, die man leicht an der Verbreiterung des Vorfußes erkennt.

Vom pes supinatus spricht man, wenn bereits in Ruhestellung der innere Fußrand höher steht als der äußere, wodurch sodann der vordere Fußrandteil an der Innenseite (medial) weniger und an der Außenseite (lateral) vermehrt belastet wird. Korrigiert man nun durch die bisher bekannte Knick-Plattfuß-Einlagen die X-Stellung der Ferse, dann wird, insbesondere beim Vorliegen eines pes supinatus, die Außenrandbelastung des Vorfußes verstärkt, so daß der innere (mediale) Fußrand keinen oder nur verminderten Bodenkontakt hat. Daran ändert auch die sogenannte Detorsionseinlage nach Hohmann nichts, welche eine Erhöhung des vorderen äußeren Randes hat. Mit der Erfindung wird demgegenüber nun vorteilhafterweise bei einer Knick-Plattfuß-Korrektur gleichzeitig dafür gesorgt, daß der Vorfuß aus seiner schädlichen Supinationsstellung herausgebracht und entsprechend gedreht (verwrungen) wird. Bei dieser Drehbewegung wird außerdem das Längsgewölbe verstärkt, d. h. die Senkfuß-Deformität vermindert. Dies gilt insbesondere wenn der innere Fußrand im Bereich unmittelbar vor der Ferse durch das Einlagen-Längsgewölbe abgestützt wird. An dieser Stelle wird ferner darauf hingewiesen, daß die mediale Absenkung des Vorfußbereiches beim Verdrehen oder Verwringen gemäß Anspruch 1 den zusätzlichen Vorteil einer gleichzeitigen Spreizfuß-Korrektur beinhaltet, d. h., das Quergewölbe des Vorfußes wird verstärkt wenn der innere Rand des Vorfußes — wie beschrieben — gesenkt gleichzeitig aber sein mittlerer Anteil durch eine sogegenannte Spreizfußpelotte von der Sohle her abgestützt wird.

Ferner ist mit der Erfindung eine Behandlung des sogenannten pes adductus (Sichelfuß) möglich. Dies ist eine Fußdeformität, bei welcher der vordere Abschnitt des Fußes zu stark nach innen gebogen ist. Hiermit zusammen tritt zumeist ein pes supinatus (siehe oben) auf. Diese beiden letztgenannten Deformitäten sind die am häufigsten auftretenden Teilkomponenten des Klumpfußes, der ein überwiegend angeborenes Leiden ist. Seine bisherige Behandlung mittels sogenannter Hilfsmittel war nur durch Schuh-Einlagen, Schuh-Zurichtungen, dem speziellen « Anti-Varus »-Schuh und vielfachen schienenähnlichen Orthesen möglich, wobei aber immer auf den Fuß ein ständiger, korrigierender Druck ausgeübt wurde. Nachteilig sind hierbei die Druckbeschwerden, Zirkulationsstörungen, psychische Einflüsse und anerkannterweise der relativ schlechte therapeutische Erfolg.

Insgesamt wird mit der Erfindung sowohl bei der Behandlung des Knick-Plattfußes, als auch bei der Behandlung des Sichelfußes und des pes supinatus vor allem aufgrund der enormen Vielzahl der einzelnen, intermittierenden Wirkungseinflüsse beim Gehen, ein medizinisch besseres Ergebnis als bei den bisher bekannten konservativen Behandlungen erreicht. Dies auch nicht zuletzt durch den Vorteil einer wesentlich besseren Verträglichkeit gegenüber den bisher üblichen Hilfsmitteln. Als weiterer Vorteil kommt hinzu, daß deises orthopädische Schuhwerk oder diese Schuhwerkteile nach der Erfindung mit einem im Verhältnis geringen konstruktiven und fertigungstechnischen Aufwand herstellbar sind. Insbesondere sind die hiermit entstehenden Kosten in keiner Weise mit den demgegenüber um ein Vielfaches höheren sonstigen Kosten — insbesondere Operationen und heilgymnastischen Behandlungen — zu vergleichen.

Die Erfindung erlaubt auch eine sehr einfache Korrektur der X-Stellung der Großzehe (Hallux-Valgus) zusammen mit der gleichzeitig auftretenden Deformation der Zehen zwei bis fünf in Form sogenannter Krallenzehen bzw. Hammerzehen.

Die Erfindung bezieht sich ferner auf eine Reihe von Anordnungen und Mittel, die den entsprechenden Fußteilen die jeweils gewünschte Korrekturbewegung beim Gehvorgang erteilen. Dies können Gestänge oder in bevorzugter Ausführungsform der Erfindung Rollen oder Gleitflächen sowie Bänder oder Seile oder auch ein hydraulisches Kammersystem sein. Hierzu wird auf die Unteransprüche verwiesen.

Weitere Einzelheiten der Erfindung ergeben sich aus der nachstehenden Beschreibung und der zugehörigen Zeichnung von erfindungsgemäßen Ausführungsbeispielen. In der Zeichnung zeigt :

Figur 1 eine Draufsicht auf ein erstes Ausführungsbeispiel der Erfindung für die Behandlung eines Knick-Plattfußes mit Vorfußsupinationsstellung,

Figur 2 einen Schnitt gemäß der Linie II-II in Fig. 1,

Figur 3 einen Schnitt gemäß der Linie III-III in Fig. 1,

Figur 4 einen Schnitt gemäß der Linie IV-IV, in Fig. 1,

Figur 5 in einer Draufsicht ein weiteres Ausführungsbeispiel der Erfindung zur Behandlung des Sichelfußes und gleichzeitig evtl. auch bestehenden pes supinatus,

Figur 6 eine Seitenansicht auf Fig. 5 gemäß dem Pfeil VI,

Figur 7 ein weiteres Ausführungsbeispiel der Erfindung zur Behandlung des Sichelfußes und/oder des pes supinatus,

Figur 8 eine Seitenansicht zu Fig. 7 gemäß dem Pfeil VIII in Fig. 7,

Figur 9 einen Schnitt gemäß der Linie IX-IX in Fig. 7,

Figur 10 ein weiteres Ausführungsbeispiel zur Behandlung des Hallux-Valgus und der Hammerzehen in der Draufsicht,

Figur 11 eine Draufsicht gemäß Fig. 10, unter Weglassung der die Zehen abdeckenden Teile,

Figur 12 die Seitenansicht zu Fig. 10 in Richtung des Pfeiles XII,

Figur 13 einen Schnitt gemäß der Linie XIII-XIII in Fig. 11.

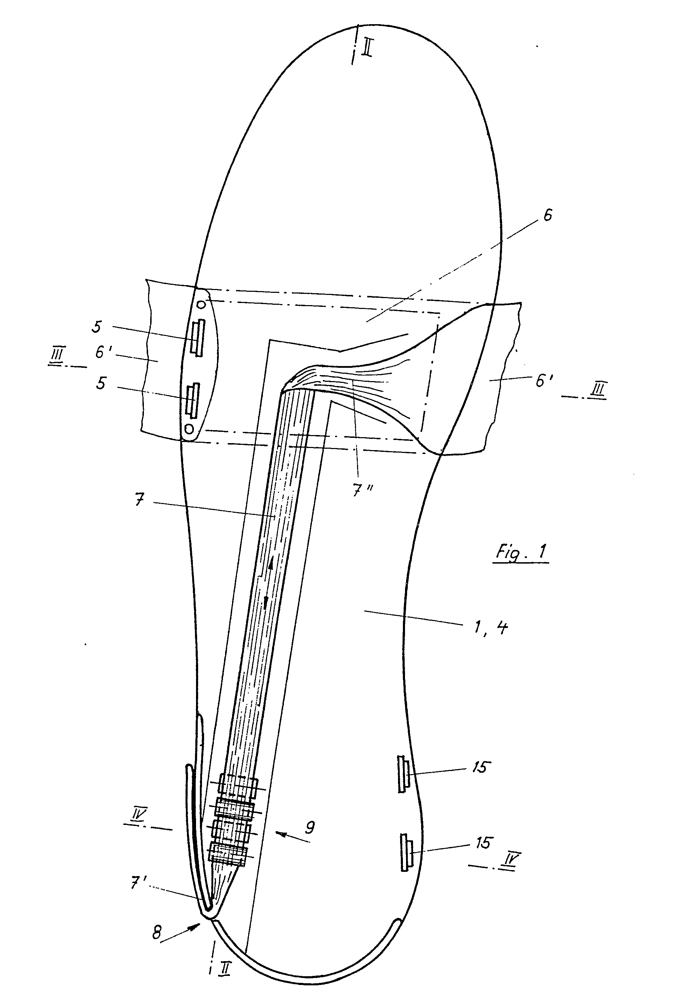

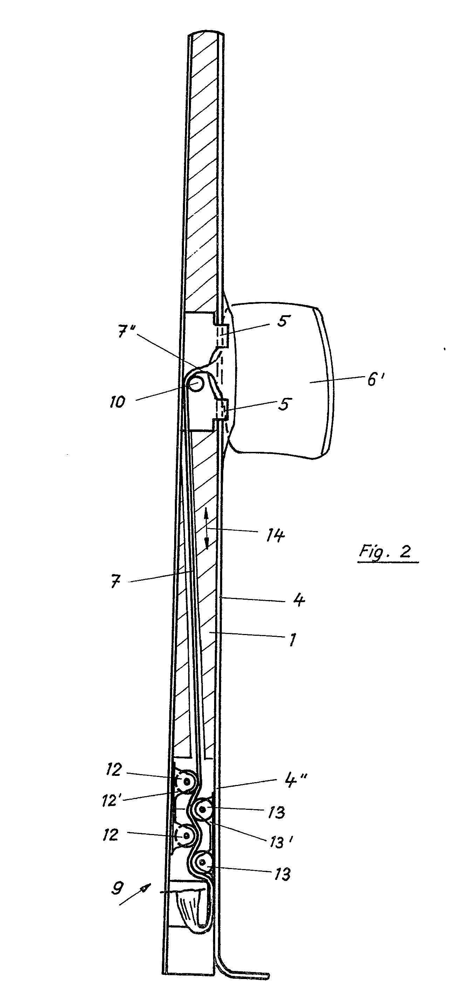

Das Beispiel der Fig. 1 bis 4 zeigt eine dynamische Knick-Plattfuß-Einlage. Hierdurch soll bei jeder Belastung der Ferse und insbesondere beim Gehen mit jedem Schritt die Ferse lateral und der Vorfußbereich medial abgesenkt werden. Damit ist der Drehsinn des Vorfußes gegenläufig zum Drehsinn der Ferse und es tritt der gewünschte Verwringeffekt ein. Die nur beispielhaft zu verstehende Ausführung der vorgenannten Figuren ist im einzelnen wie folgt :

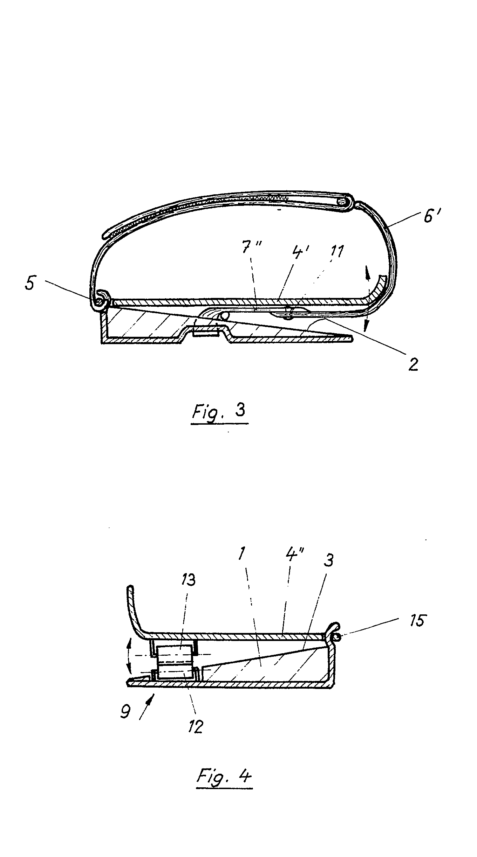

Der Grundkörper 1 einer Einlage besteht beispielsweise aus einem elastischen Kork von einer Stärke von etwa 1 cm. Wie Fig. 3 zeigt, ist der Vorfußteil des Einlagenkörpers an seiner Oberseite 2 für die zu erreichende Pronationsstellung (mediales Absinken) des Fußes geneigt, z. B. 15°.

An dieser Stelle sei vermerkt, daß mit dem Begriff « lateral » immer die nach außen gelegene Längsseite und mit dem Begriff « medial » immer die nach innen gelegene Längsseite des Fußes gemeint ist.

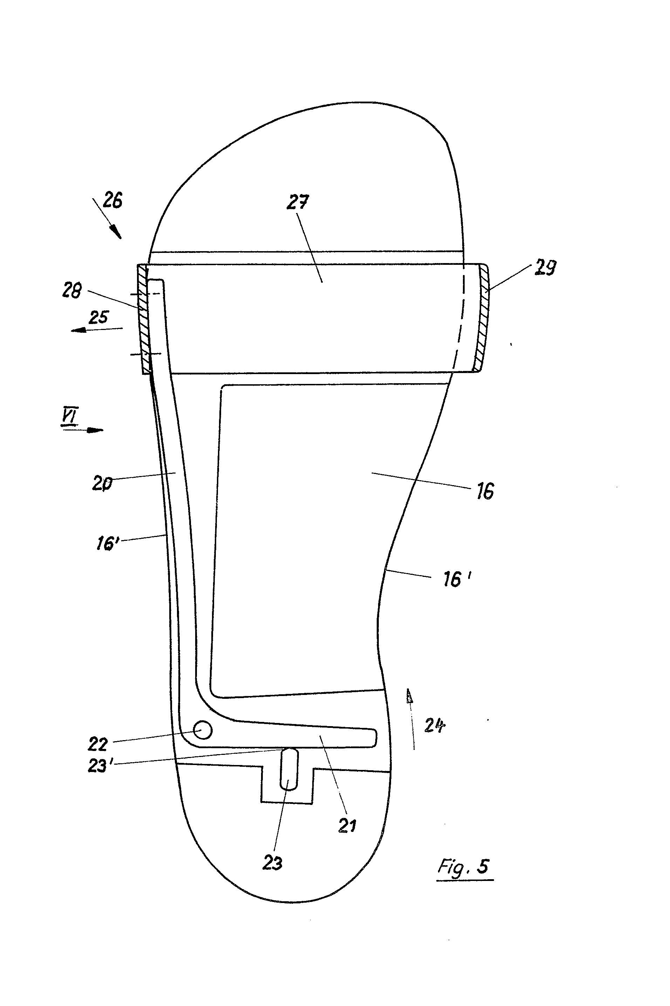

Dagegen ist gemäß Fig. 4 der Fersenteil des Einlagenkörpers für die Erreichung der Supinationsstellung (laterales Absinken) so geneigt, daß die Belastungsfläche 3 nach außen (lateral) absinkt, z. B. ebenfalls mit einem Winkel von 15° zur Horizontalen. Oberhalb des Einlagenkörpers 1 und den Flächen 2, 3 liegt eine elastische Auftrittsfläche 4 der Einlage, die z. B. aus einem Kunststoff (z. B. bekannt unter dem Warenzeichen Ortolen) bestehen kann. Die Auftrittsfläche 4 ist mittels einer gelenkähnlichen Aufhängung, z. B. einem Steckgelenk 5 vorn an der lateralen Seite der Ballenauftrittsfläche 6, sowie mittels einer ähnlichen Aufhängung bzw. Steckgelenk 15 im Fersenbereich an der medialen Seite der Sohle schwenkbar befestigt (oder es wird anstelle dieser Gelenke ein Material von einer geeigneten Biegbarkeit und Festigkeit verwendet). Der Einlagenkörper kann speziell im Bereich der Armierungen 5, 15, 12 und 13 (siehe nachstehende Beschreibung) beispielsweise durch Kunststoff oder Leichtmetall stabilisiert sein. Der Riemen 6' dient zur Verbindung zwischen der Auftrittsfläche und dem Vorfuß und läßt sich über dem Fußrücken stufenlos in seiner Länge verstellen und schließen (z. B. Klettenverschluß). Er ist entweder an den beiden Rändern der Sohle 4' befestigt (nicht dargestellt) oder aber gemäß einer bevorzugten und in der Zeichnung (siehe insbesondere Fig. 3) dargestellten Ausführungsform lateral an der Sohlenkante befestigt, während der mediale Riemenanteil 6' anstelle einer Befestigung am Sohlenrand über eine Gleitfläche des Randes der Belastungsfläche 4′ verlaufend direkt in das Zugband 7″ übergehen kann, was den Vorteil eines funktionell intensiveren Kontaktes des Vorfußes mit seiner Auftrittsfläche und damit eine stärkere Korrekturwirkung auf den pes supinatus und Spreizfuß hat. Ein bevorzugt aus Kunststoff bestehendes Band oder Seil 7 ist an der lateralen Seite des Absatzes 8 verstellbar befestigt (siehe Ziffer 7′) und läuft von dort durch den hinteren Absatzrand und eine noch näher zu erläuternde Flaschenzugmechanik 9 nach vorn zu einer Rolle oder Gleitfläche 10, die etwa unter der Mitte des Vorfußballens oder etwas seitlich davon liegt. Dort wird das Band oder Seil 7 nach oben umgelenkt (Ziffer 7″). Es kann an der Unterseite des um das Gelenk 5 kippbaren Teiles 4′ der Auftrittsfläche 4 befestigt sein (nicht dargestellt). In der bevorzugten und in der Zeichnung dargestellten Ausführungsform ist dieser Teil 7″ des Bandes gemäß Ziffer 11 mit dem medialen Riemen 6′ verbunden (siehe insbesondere Fig. 3). Die Spannung des Bandes oder Seiles 7 ist sodann sowohl bei seiner Befestigung lt. Ziffer 7′ als auch mittels der Vorfußriemen 6′ einstellbar. Es kann eine Sicherung dagegen vorgesehen sein, daß das Band 7 versehentlich nach einer der beiden Seiten zu weit herausgezogen wird. Dies gilt sinngemäß auch für die übrigen Bänder der anderen Ausführungsbeispiele.

Die obengenannte « Flaschenzug »-Mechanik besteht hier aus zwei im Absatzteil des Einlagenkörpers 7 fest angebrachten (Ziff. 12′) Rollen oder Gleitflächen 12 und zwei an der Unterseite der beweglichen Auftrittsfläche 4 bzw. 4″ angebrachten (Ziff. 13′) Rollen oder Gleitflächen 13, wobei zwischen den Rollen 12, 13 das Band oder Seil 7 hindurchgeführt ist. Es ist ersichtlich, daß mit einem Absenken (Anheben) der Fläche 4″ um die Länge x das Band oder Seil 7 in der Funktionsrichtung gemäß Pfeil 14 um den Betrag 4 x in Richtung zur Ferse (von der Ferse weg) sich bewegt. Das erläuterte Flaschenzugprinzip kann auch gegebenenfalls mit mehr oder weniger Rollen für die anderen, in dieser Beschreibung erläuterten intermittierenden Korrekturen von Fußdeformitäten verwendet werden, wie es sich z. T. aus den Zeichnungen ergibt. Bei der Plattfuß-Korrektureinlage gemäß den Fig. 1-4 ist die Lagerung dieses Flaschenzug-Mechanismus speziell im lateralen hinteren Absatzbereich dann sinnvoll, wenn im Moment der Fersenbelastung eine Kippbewegung der Ferse im Sinne einer Supinationsbewegung gewünscht wird (Korrektur des Knickfußes). Für diesen Fall ist die halbelastische nachgiebige Fersenauftrittsfläche 4″ an ihrem medialen Rand scharnierähnlich zu lagern (Ziff. 15) und der darunterliegende Absatzanteil keilförmig zu unterhöhlen (Fig. 4). Die Einlage soll in speziellen Einlagenschuhen oder Sandalen mit elastisch biegbarer Sohle getragen werden, welche dem Vorfuß die erforderliche Höhe für die Verwringbewegung bieten und eine gute Halterung des Mittel- und Rückfußes im Schuh ermöglichen. Der Fersenmechanismus (4″, 12, 13) tritt in Funktion, sobald die Einlage im Fersenabschnitt belastet wird. Sodann gleiten die an der unteren Seite der Einlage befestigten Rollen 13 unmittelbar hinter den im Absatz stabil befestigten Rollen 12 nach unten vorbei und bringen dabei das Zugseil 7 in Spannung. Hierdurch wiederum wird der mediale Vorfußabschnitt einschließlich dem medialen Vorderanteil 4′ der Einlage nach unten gezogen (der Vorfuß proniert). Eine ausreichend feste Verbindung (siehe die obengenannte Mittelfußbandage 6′) des Vorfußes mit dem vorderen Einlagenabschnitt ist Bedingung für die gewünschte pronatorische Drehbewegung. Durch eine gleichzeitig vorgesehene ausreichend stabile Halterung auch des Rückfußes im Bereich der nach außen schräg abfallenden Fersenauftrittsfläche 4″ kommt es — vor allem bei geeignet geformter Einlagen-Auftrittsfläche — während des funktionellen Ablaufes zu der gewünschten « Verwringung » des Fußes, die solange anhält, wie der Rückfuß belastet wird. Die Freigabe des Fußes aus dieser Verwringbewegung erfolgt bei der Entlastung, d. h. im Gehen beim Ende der Abrollphase. Durch die Verwringung des Fußes mittels dieser Einlage wird zusätzlich zu der Korrektur des Knickfußes und des pes supinatus auch das Längs- und Quergewölbe des Fußes verstärkt, d. h. die Senk- und Spreizfußdeformität korrigierend beeinflußt. Die jeweiligen funktionellen Bewegungsrichtungen sind durch Pfeile eingezeichnet.

Die gesamte Anordnung läßt sich, wie bereits erwähnt, als Einlage in speziell ausgebildeten Schuhen von entsprechender Innenhöhe lose einlegen. Auch ist es möglich, die Anordnung als Schuhwerk, d. h. Schuhe oder Sandale, auszubilden.

Das Anspannen des Zugseiles oder -bandes 7 kann auch durch die Umfangsvergrößerung einer flachen, etwa z. B. linsenförmigen Kammer (z. B. Gummi- oder Plastikballon) erfolgen, deren Wände verformbar sind, deren Inhalt aber flüssig und damit nicht komprimierbar ist, so daß das Gesamtvolumen sich bei der Kompression nicht verändert. Um die kreisrunde Circumferenz verläuft in einer Halterung gleitbar gelagert das Zugseil. Bei der Fersenbelastung wird die Kammer etwa in vertikaler Richtung komprimiert, so daß sich ihre Wände nur in radialer Richtung ausdehnen können, wodurch sich der kreisförmige Umfang vergrößert und das dort gelagerte Zugseil anspannt (nicht dargestellt).

Anstelle der supinatorischen Kippbewegung der Ferse kann ein ähnlicher Effekt auch durch einfache Senkbewegung der Ferse bei entsprechend vereinfachtem Mechanismus ausgelöst werden, wobei die Fersenauftrittsfläche der Einlage sich bei ihrer Belastung ebenfalls in Richtung zum Absatzteil der Einlage senkt, wie dies auch in der Beschreibung des ersteren Modelles der Fall ist (nicht dargestellt).

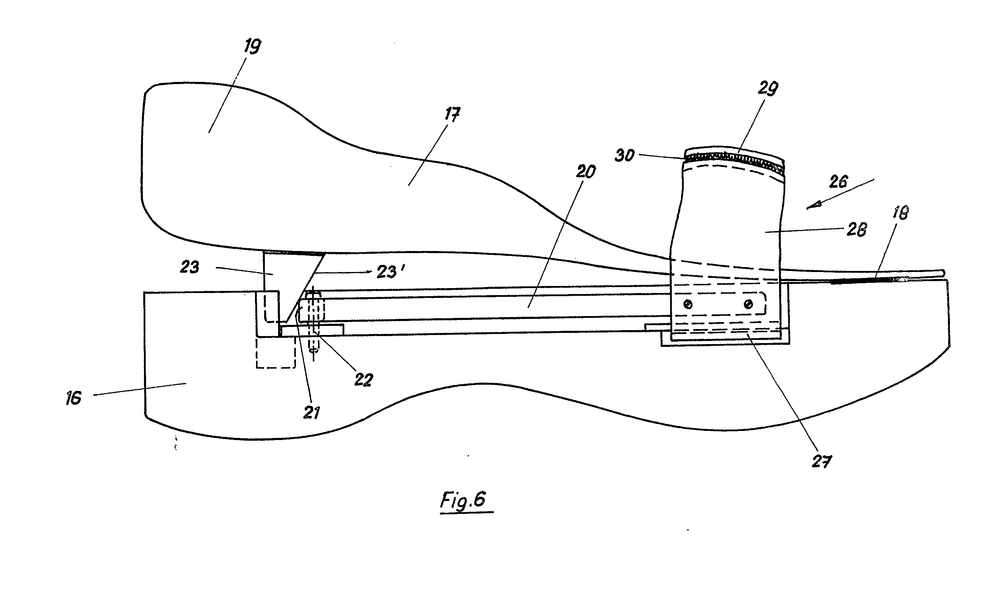

Im Ausführungsbeispiel der Fig. 5 und 6 ist eine Sandale zur Behandlung des Sichelfußes (pes adduktus) in Verbindung mit einer Behandlung des pes supinatus dargestellt. Die Sandalensohle 16 trägt eine Schaleneinlage 17 für das Aufsetzen des Fußes, die aus Gründen der zeichnerischen Darstellung des zu erläuternden Hebelmechanismus in Fig. 5 nicht gezeichnet ist. Die Schaleneinlage 17 ist nur im Bereich des Vorfußes querverlaufend an der Sohle 16 fixiert (siehe Ziff. 18), während sie in ihrem übrigen Bereich leicht angehoben werden kann. Die Anhebung kann am Fersenteil 19 bis 3 cm betragen. Ein aus den beiden Armen 20, 21 bestehender Hebel ist um die vertikale Achse 22 zur Sohle 16 verschwenkbar. Zu diesem Zweck ist der bevorzugt kürzere Hebelarm 21 im Absenkbereich eines Vorsprunges 23 der Unterseite des Schalen-Fersenteiles 19 gelegen. Mit dem Absenken dieses Fersenteiles wird über die schräg verlaufende Kante 23' des Tuiles 23 der Hebelarm 21 in Richtung des Pfeiles 24 bewegt, so daß der bevorzugt längere Hebelarm 20 in Richtung des Pfeiles 25 gedrückt wird. Die in Richtung des Pfeiles 25 wirkende Kraft hat nicht nur die Korrektur des pes adduktus (Sichelfuß), sondern auch des zugehörigen pes supinatus, d. h. ein Drehen des Vorfußbereiches in der Weise zur Folge, daß er medial absenkt. Dies erfolgt mit Hilfe eines Doppelriemens oder einer Schlaufe oder eines Zügels 26, der bzw. die mit ihrem unteren Abschnitt 27 in einem Tunnel der Sohle 16 unter einer darüber befindlichen stabilen Platte verläuft und mit den beiden oberen Enden 28, 29 (in Fig. 5 nur teilweise dargestellt) oberhalb des Fußes mit Hilfe eines Verschlusses 30 zusammengehalten wird. Dabei wird der Fuß etwa im Bereich der vorderen Hälfte des ersten Mittelfußknochens einschließlich dem Grundglied der Großzehe umfaßt. Der Hebelarm 20 greift mit seinem vorderen Ende am seitlichen unteren Schaufenteil 27 an, so daß seine Bewegung in Richtung des Pfeiles 25 neben der Sichelfuß-Korrektur auch die erläuterte Drehbewegung des Vorfußes im Sinne der Pronationsbewegung bewirkt, zumal die mediale Öffnung des Tunnels wegen der günstigeren Zugrichtung tiefer unten in der Sohle liegt als die laterale. Diese Neigung des Tunnels ist zeichnerisch nicht dargestellt. Sie empfiehlt sich, falls mit der Korrektur des pes adduktus auch die des zugehörigen pes supinatus erfolgen soll. Dabei ist die Oberfläche der Sohle 16 so gestaltet, daß zwischen dem medialen Rand des Vorfußes und diesem Oberflächenteil der Sandalensohle ein das vorgenannte Absenken ermöglichender freier Raum besteht. Nach der Freigabe des Fersenteiles 19 vom Fußdruck geht der Fuß aufgrund seiner Fehlstellung von selbst wieder in die Ausgangslage zurück. Im Rückfußbereich wird er durch einen geteilten Riemen an den Rändern der Einlagenschale gehalten.

Wie die Fig. 5 zeigt, steht zweckmäßigerweise der kurze Hebelarm 21 in seiner durch den Vorsprung 23 nicht verschwenkten Lage etwa quer zur Längsachse der Sandale. Das Übersetzungsverhältnis vom kurzen 21 zum langen Hebelarm 20 kann etwa 1 : 5 betragen, so daß sich der vom Fersenteil 19 über den Vorsprung 43 ausgeübte Bewegungsausschlag am vorderen Ende des langen Hebelarmes 20 verfünffacht.

Der Grundgedanke dieses Teiles der Erfindung, nämlich durch ein Absenken der Ferse sowohl die Sichelfuß-Korrektur als auch die des pes supinatus vorzunehmen, kann auch in anderer Weise verwirklicht werden, z. B. kann der Fersenteil direkt oder über einen dem Vorsprung 43 ähnlichen Teil einen Kniehebel oder ein Scherengittersystem (nicht dargestellt) betätigen, das die Kraft auf einen gemäß Pfeil 25 lateralen Zug an der Schlaufe 26 überträgt. (analog Fig. 5).

Auch kann eine Hebelanordnung so gebildet sein, daß sie eine ausreichend breite, gepolsterte Druckplatte gegen die innere, obere Seite des Vorfußes drückt und damit den Vorfuß in Richtung Korrekturstellung bewegt.

Die Zugkraft könnte auch mit Hilfe eines Seiles ausgeübt werden, das über eine Umlenkrolle die Schlaufe 26 in Richtung des Pfeiles 25 zieht. Die Zugkraft des Seiles geht von einer kurzen Welle aus, die über ein Steilgewinde im Absatz gelagert ist, wobei die Welle durch den Druck des Fersenteiles 19 nach unten gedrückt wird. Aufgrund der Steilgewinde-Lagerung erhält die Welle dabei eine Rotationsbewegung. Damit wird das am Umfang der Welle angebrachte Seil entsprechend gezogen.

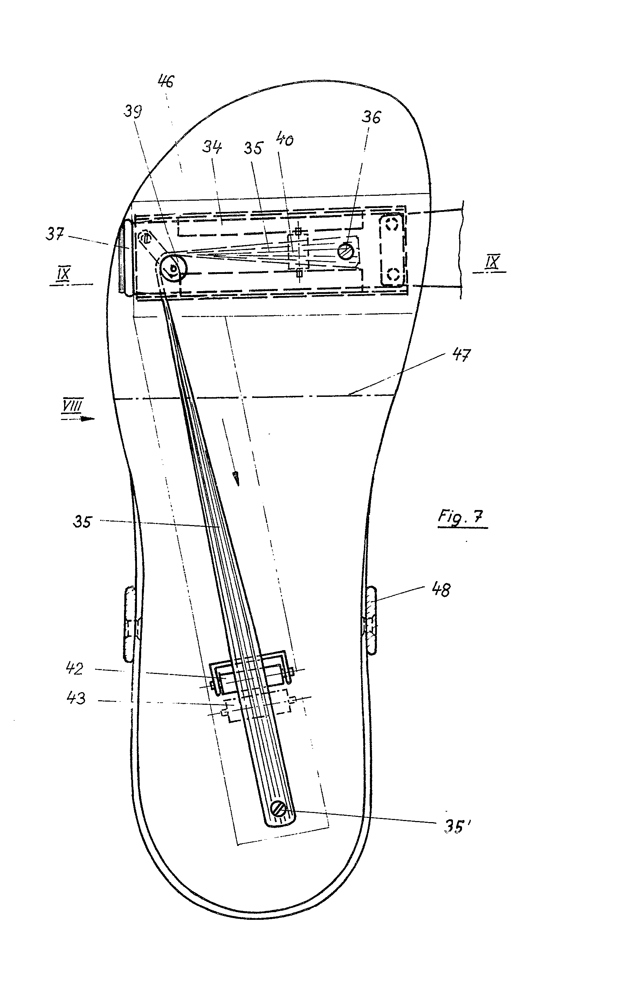

Anstelle des unteren Schlaufenteiles 27 des Ausführungsbeispieles der Fig. 5 und 6 könnte dort auch ein in einer entsprechenden Ausnehmung des Vorfußbereiches der Sohle verschiebbar gelagerter Schieber vorgesehen sein, der vom längeren Hebelarm 20 nach lateral verschiebbar ist. Eine mögliche Ausführungsform eines solchen Schiebers ist dem später erläuterten Ausführungsbeispiel gemäß Fig. 7-9 zu entnehmen.

Das Ausführungsbeispiel der Fig. 7 bis 9 zeigt eine dynamische Sichel-Klumpfuß-Sandale, deren Aufbau wie folgt ist :

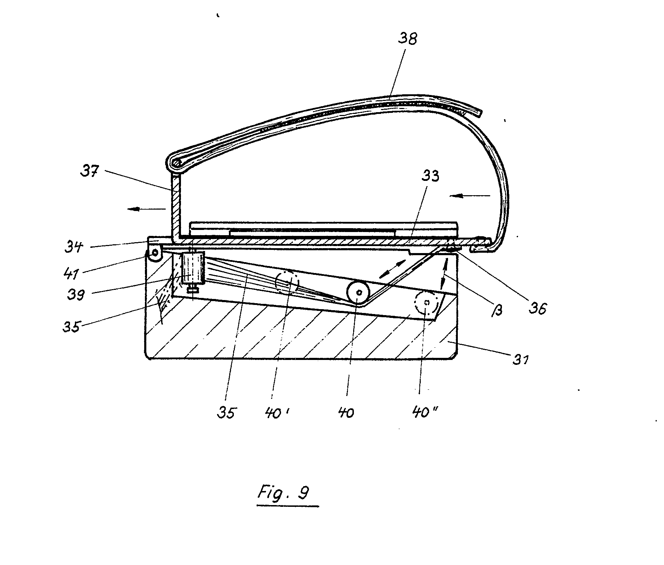

Die stabile Sohle (z. B. Kunststoff oder Holz) erlaubt dem vorderen Fußbereich gemäß Fig. 9 eine durch Keilauflagerung veränderliche Pronationsstellung der Vorfußauftrittsfläche 33 bis zu einem Winkel β von z. B. etwa 15°. In diesem vorderen Fußbereich ist die Sohle 31 mit einer breiten Rille 34 versehen, welche den in der Zeichnung waagerechten Teil des Schiebers 33 mit Schiebe- oder Gleitsitz aufnimmt. Ferner kann in dieser Rille 34 das Seil oder Band 35 verlaufen, das bei 36 am Schieber angebracht ist und sich von dort her nach lateral sowie nach unten erstreckt. Der Schieber besitzt ferner an einem seiner Enden (dies ist gemäß diesem Ausführungsbeispiel das laterale Ende, es könnte aber auch das mediale Ende sein) einen in Höhe des jeweiligen Vorfußrandes etwa senkrecht nach oben verlaufenden Fortsatz 37. Der Vorfußriemen 38 ist am medialen Rand des Schiebers und am lateralen Rand (hier am Fortsatz 37) angebracht und z. B. mittels eines Klettverschlusses verstellbar. Die Spannung dieser dann nur noch semicirkulär wirkenden Schlaufe ist also stufenlos einstellbar. Eine cirkulär wirkende Einschnürungsgefahr besteht nicht.

Das Band oder Seil 35 läuft von seinem Punkt 36 entweder nur parallel zum Schieber 33 und über die Rolle 39 nach hinten zur Fersenbetätigung. In diesem Fall hat eine Bewegung des Bandes oder Seiles eine reine Vorfußabduktionsbewegung (Bewegung des Vorfußes waagerecht nach lateral) zwecks Sichelfußkorrektur zur Folge. Man kann des Band oder Seil 35 aber auch um eine Rolle 40 laufen lassen, die gemäß Ziffer 40' und 40" an verschiedenen Stellen der Sohle einsetzbar ist. Bei der Lage gemäß Ziffer 40" hat ein Ziehen des Bandes oder Seiles 35 praktisch nur noch ein Schwenken des Schiebers 33 mit seiner bei 41 gelagerten, ihn aufnehmenden Hülle oder Schiene um diesen Punkt in Richtung des Winkels β nach unten zur Folge. In diesem Fall besteht die Sandalenfunktion aus einer im wesentlichen reinen Vorfußpronationsbewegung (mediales Absenken des Vorfußes) zwecks pes supinatus-Korrektur. Nimmt die Rolle eine der Positionen 40 bzw. 40' ein, so wird ein Ziehen des Bandes 35 durch Betätigung des Fersenmechanismus eine Mischung aus einer Vorfußpronationsbewegung und einer Vorfußabduktionsbewegung zur Folge haben (Klumpfußkorrektur mit verschiedener Stärke der Teilkomponenten).

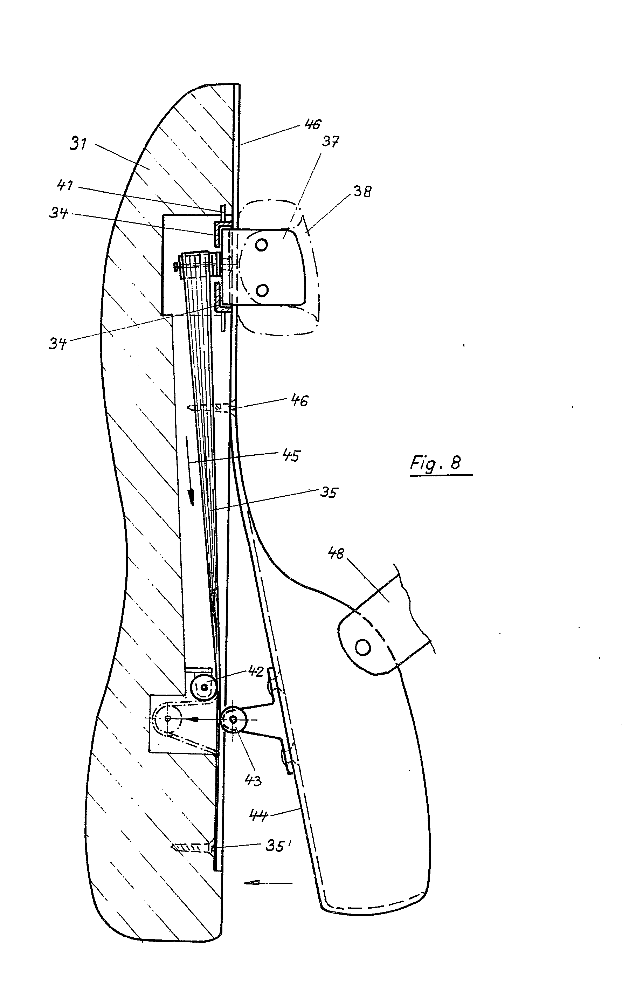

Der Verlauf des Bandes oder Seiles 35 vom Schieber zur Fersenbetätigung ist am besten den Fig. 7, 8 zu entnehmen. Es läuft in einer Längskerbe der Sohle zum Absatz, wo es gemäß Ziffer 35' an der Sohle angebracht ist. Diese Anbringung kann verstellbar und in der jeweiligen Lage des Bandes fixierbar sein, so daß hiermit die Vorspannung des Bandes änderbar ist. Dies gilt sinngemäß auch für die Bandbefestigungen der anderen Ausführungsbeispiele, wie überhaupt bei einem der Ausführungsbeispiele dargestellte Anordnungen und Mittel sinngemäß auch bei einem der anderen Ausführungsbeispiele verwendet werden können und umgekehrt.

Der auslösende Fersenmechanismus besteht hier aus zwei parallel gelagerten Rollen (oder entsprechenden Gleitflächen), von denen die vordere Rolle 42 an der Sohle und die rückwärtige Rolle 43 an der beweglichen Auftrittsfläche 44 angebracht ist. Das Band oder Seil 35 läuft so durch die Rollen hindurch, daß hier ebenfalls das obengenannte Flaschenzugprinzip wirksam wird und zwar mit einer Übersetzung der Niederbewegung der Auftrittsfläche 44 in eine Bewegung des Seiles in Richtung des Pfeiles 45 um die doppelte Weglänge.

Die Auftritts- oder Belastungsfläche 44 kann aus einer schalenförmigen Einlage (z. B. Gießharz) bestehen, die im Vorfußbereich gemäß Ziffer 46 fest mit der Sohle 31 verbunden ist und beim Aufsetzen des Hackens um eine horizontal und quer zur Fußlängsrichtung verlaufende Achse verschwenkt wird, die in Fig. 7 mit Ziffer 47 strichpunktiert angedeutet ist. Der vordere feste Teil 46 der Auftrittsfläche 44 deckt oberseitig die Rille 34 zu einem Tunnel ab. Der bis zum Rückfuß reichende äußere Schalenrand der Auftrittsfläche 44 ist kräftig und an seiner Innenseite gut gepolstert. Der hintere Fußabschnitt wird dort mittels eines breitflächigen Riemens 48 in einer für Sandalen üblichen Form gehalten.

Erwähnt sei, daß durch die oben schon genannte individuell einstellbare Spannung des Vorfußriemens 38 erreicht wird, daß bei jeder Zugbewegung auf das Band oder Seil 35 der Vorfuß wie erläutert im Sinne einer Abduktions- und/oder Pronationsbewegung bewegt wird. Diese Korrekturbewegung kann durch eine entsprechende Vorspannung des Vorfußriemens 38 verstärkt, oder bei empfindlichen Füßen auch abgeschwächt werden.

Wie bereits erläutert kann die Bewegung eines Schiebers gemäß Ziffer 33 auch von einem Hebel her erfolgen, z. B. dem Hebelarm 20 im Beispiel der Fig. 5, 6.

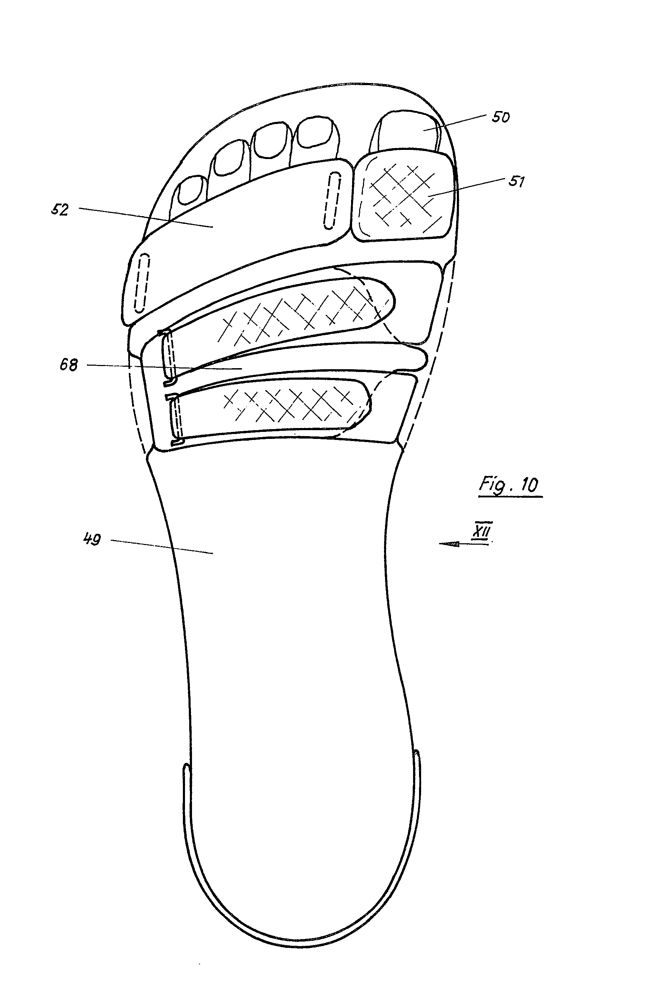

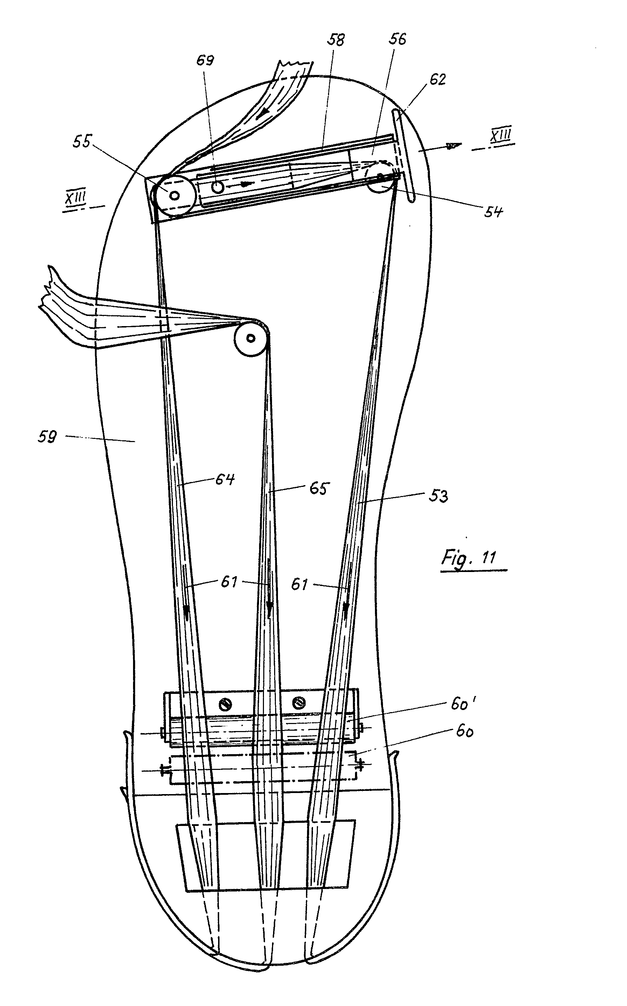

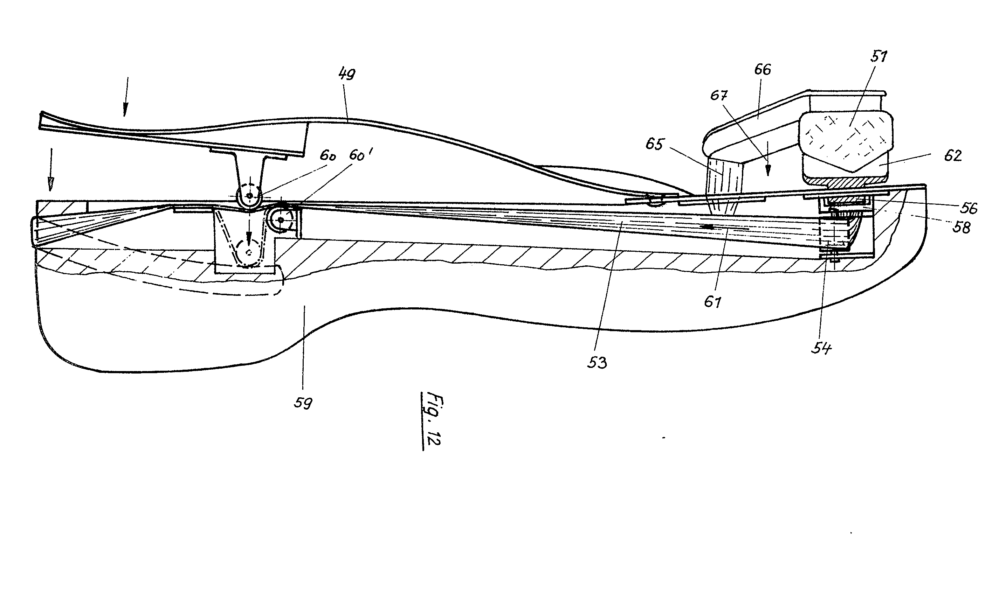

Das Ausführungsbeispiel der Fig. 10 bis 13 zeigt die Ausgestaltung eines Schiebers, der in vereinfachter Form ähnlich dem Schieber 33 des vorhergehenden Ausführungsbeispieles ist, jedoch nicht wie beim vorhergehenden Beispiel auf den gesamten Vorfuß einwirkt, sondern nur zur Korrektur der X-Stellung der Großzehe (Hallux-Valgus) Verwendung findet. Fig. 10 zeigt im Prinzip die Draufsicht mit der Auftrittsfläche 49, einer die Großzehe 50 umgebenden Schlaufe 51 und einer Druckplatte 52 zur Korrektur der Hammerzehenstellung der übrigen Zehen Nr. zwei bis fünf.

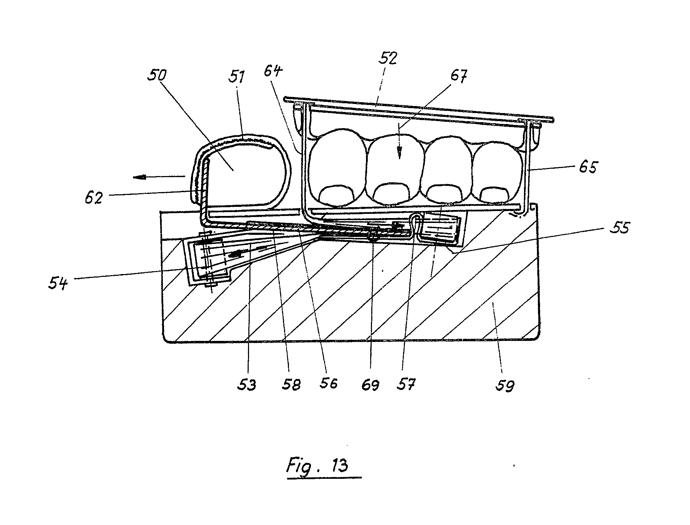

Für die Korrektur der X-Stellung der Großzehe ist ein Band oder Seil 53 vorgesehen, das auf einer Rolle oder Gleitfläche 54 läuft und ziemlich lateral gemäß Ziffer 55 zuerst an der Unterseite des Schiebers 56 gemäß Ziffer 69 befestigt und dann unmittelbar lateral davon gemäß Ziffer 55 an der Sohle 59 angebracht ist. Das Band 53 unterläuft also gemäß Fig. 13 den Schieber 56 und bildet zwischen seinen beiden Festpunkten 69 und 55 eine schlaufenförmige Falte 57 und zwar nur in der Ausgangsstellung des Schiebers 56, während das Band bei der extremen Endstellung des Schiebers (extreme Verschiebung nach medial) sich in Spannung befindet. Durch die Befestigung des Bandendes an der Sohle 59 wird verhindert, daß der Schieber beim Anspannen des Bandes zu weit nach medial herausgezogen wird.

Der Schieber 56 läuft in einer U-förmigen Führungsschiene 58, in deren Bodenfläche eine größere Öffnung für den Durchtritt des Bandes 53 besteht. Die obengenannte Rolle 54 ist im medialen Bereich der Führungsschiene 58 angeordnet.

Wird durch Niedertreten der Fersenauflagefläche 49 die Rolle oder Gleitfläche 60, die an dessen Unterseite angebracht ist, nach unten abgesenkt, so gibt dies im Zusammenwirken mit der Rolle 60' der Sohle 59 ein Ziehen auf das Band 53 in Richtung des Pfeiles 61 und damit ein Verschieben des Schiebers 56 nach medial. An einem nach oben gerichteten Fortsatz 62 des Schiebers 56 ist eine in ihrer Länge verstellbare (z. B. mittels eines Klettverschlusses) Halteschlaufe 51 vorgesehen, welche die Großzehe 50 umgibt. Hiermit wird die Großzehe nach medial bewegt, wobei der Teil 62 dafür sorgt, daß die Großzehe nicht von der Schlaufe 51 eingeschnürt wird. Die vorstehend beschriebene Anordnung zeichnet sich durch ihre besondere konstruktive Einfachheit aus und ergibt eine optimale Lösung des nach medial gerichteten Schlaufenzuges ohne aufwendige, bzw. störende konstruktive maßnahmen am medialen Schuhrand.

In Verbindung mit dieser Hallux-Valgus-Korrektur können zwei weitere Bänder 64 und 65 vorgesehen sein, die ebenfalls von der Betätigung 60, 60' in Richtung der Pfeile 61 beweglich sind und an der Hammerzehendruckplatte 52 angreifen und diese bei der obengenannten Bewegung in Pfeilrichtung 61 gemäß Pfeil 67 nach unten auf die Zehen zwei bis fünf drücken, wodurch diese in ihren zu stark gekrümmten Gelenken gestreckt werden. Auch diese, zuletzt beschriebene Anordnung ist einfach, da mit nur zwei Bändern sämtliche Zehen zwei bis fünf im Sinne einer Krallen- zehen- oder Hammerzehenkorrektur bewegbar sind.

Sämtliche Bänder 53, 64 und 65 sind in der jeweiligen Lage einstellbar und fixierbar, wodurch die gewünschte Vorspannung erreichbar ist. Eine solche kontinuierliche Ruhespannung addiert sich zu der funktionsbedingten dynamischen Anspannung der gesamten Vorrichtung bei Abwärtsbewegung des Fersenbereiches der Auftrittsfläche 49. Insbesondere für die Betätigung der Druckplatte 52 ist es wesentlich, daß die Bänder 64 und 65 je für sich in ihrer effektiven Länge verstellt und in der jeweiligen Stell-Lage fixiert werden können, da von Fall zu Fall unterschiedliche Korrekturen der inneren und äußeren Zehen erforderlich sind und entsprechend unterschiedliche Korrekturstrecken (Bewegen nach unten) der äußeren oder inneren Zehen zwei bis fünf erfordern.

Zur Spreizfußkorrektur dient bei der Sandale gemäß Fig. 10-13 eine Spreizfußbandage 68, die stufenlos am Fußrücken in der jeweils erwünschten Stell-Lage befestigt werden kann. Hierdurch kann man einerseits die Druckbeschwerden einer eventuellen Hallux-Valgus-Exostose umgehen und findet andererseits den gewünschten Gegenhalt, welcher durch die medial-Bewegung des Hallux-Valgus bei der Korrektur erforderlich wird. Andererseits dient diese Bandage zur Halterung des Vorfußes an der Sohle. Sofern man die beiden unteren Riemenenden der Bandage jeweils mit einem Band verbindet und diese Bänder analog den Bändern 53, 64 und 65 zu den Rollen oder Gleitflächen 60 und 60' führt, wird hierdurch auch eine intermittierende Spreizfußbehandlung möglich, die zugleich mit der Fersenbetätigung für die übrigen Fußkorrekturen erfolgt.

### Ansprüche

1. Orthopädisches Schuhwerk oder Schuhwerkteil mit einer Vorrichtung zur konservativen Behandlung von Mißbildungen des Fußes, wobei der Druck des Rückfußes auf das Schuhwerk oder Schuhwerkteil in eine intermittierende Kraftwirkung auf den Vorfußbereich umgewandelt wird, dadurch gekennzeichnet, daß unter der Kraftwirkung stehende Mittel vorgesehen sind, die den Vorfußbereich um die Fußlängsachse drehen und/oder um eine senkrecht zur Sohle verlaufende Achse zur Seite (lateral oder medial) bewegen und daß die Mittel zur Verdrehung des Vorfußbereiches an der medialen Seite des Vorfußes im Sinne deren Absenkens angreifen.

2. Schuhwerk oder Schuhwerkteil nach Anspruch 1, dadurch gekennzeichnet, daß die Drehmittel in kraftschlüssiger Verbindung mit einer bei Belastung der Ferse eine Knick-Plattfuß-Korrektur bewirkenden Anordnung steht, wobei die Drehmittel und die Knick-Plattfuß-Korrekturanordnung in ihrem jeweiligen Drehsinn aneinander entgegengesetzt gerichtet sind, d. h. ein Absenken des lateralen Fersenteiles nach unten ein Absenken des medialen Mittel- oder Vorfußbereiches nach unten bewirkt, wobei bevorzugt zumindest eine aufwärts und abwärts bewegliche Schale oder Platte für das Aufsetzen des Fußes vorgesehen ist, die durch Kraftübertragung mit dem Absenken ihres Fersenbereiches das Drehen und/oder Bewegen des Vor- oder Mittelfußbereiches bewirkt.

3. Schuhwerk oder Schuhwerkteil nach Anspruch 1 oder 2, dadurch gekennzeichnet, daß als Drehmittel ein Seil oder Band (7, 56) bzw. Seile oder Bänder dienen.

4. Schuhwerk oder Schuhwerkteil nach Anspruch 3, dadurch gekennzeichnet, daß eine medial an der Sohle (1) angelenkte (15) und hinten im Fersenbereich befindliche Auftrittsplatte (4") vorgesehen ist, die unterseitig Betätigungen (13) aufweist, unter denen das oder die Seile oder Bänder (7) durchlaufen, wobei unterhalb dieser Auftrittsplatte ein Raum für deren Schwenken nach unten vorgesehen ist und daß das Band oder Seil (bzw. Bänder oder Seile) (7) zum Mittel- oder Vorfußbereich laufen und an dem Drehmittel im jeweiligen Betätigungssinn angreifen, wobei die Betätigung des Bandes oder Seiles, bzw. Bänder oder Seile nach dem Flaschenzugprinzip erfolgt in der Art, daß eine senkrechte Abwärtsbewegung der Auftrittsplatte oder dergleichen im Fersenbereich um eine bestimmte Länge in eine um ein ganzes Vielfaches demgegenüber größere Zugbewegung des Bandes oder Seiles bzw. Bänder oder Seile übersetzt wird, wobei bevorzugt mehrere Rollen oder Gleitflächen (13) an der Auftrittsfläche oder -platte und mehrere Rollen oder Gleitflächen (12) an der Sohle angebracht sind.

5. Schuhwerk oder Schuhwerkteil nach Anspruch 4, dadurch gekennzeichnet, daß bei einer Knick-Plattfuß-Einlage die nach dem Flaschenzugprinzip gestaltete Anordnung sich im lateralen hinteren Absatzbereich unterhalb der Auftrittsplatte (4") befindet, die medial an der Sohle angelenkt ist (15) und daß ein Band oder Seil an eine im Bereich des Mittel- oder Vorfußes vorgesehene vordere Auftrittsplatte (4') an deren Unterseite mit einer etwa senkrecht nach unten gerichteten Zugrichtung angreift, oder dort nach medial abgelenkt wird und in den medialen Vorfußriemen übergeht, wobei die Auftrittsplatte (4') lateral an der Sohle angelenkt ist (5) und unter der Auftrittsplatte Raum zu deren Schwenken um die Anlenkung (5) nach unten besteht.

6. Schuhwerk oder Schuhwerkteil nach einem der Ansprüche 3 bis 5, dadurch gekennzeichnet, daß die effektive Länge des Bandes oder Seiles bzw. Bänder oder Seile zwecks Erzielung einer individuellen Spannung seine Stellung verstellbar und in der jeweiligen Stell-Lage fixierbar ist.

7. Schuhwerk oder Schuhwerkteil nach einem der Ansprüche 3 oder 6, dadurch gekennzeichnet, daß unterhalb der rückwärtigen Auftrittsplatte (4") eine, z. B. in der Form linsenförmige, hydraulische Kammeranordnung aus nachgiebigem Material mit flüssigem Inhalt vorgesehen und vom Band oder Seil umgeben ist derart, daß ein Abwärtsbewegen der Auftrittsplatte eine Ausdehnung der mit dem flüssigen Inhalt versehenen Kammer an deren Umfangslinie und damit eine Zugbewegung auf das Seil oder Band bewirkt.

8. Schuhwerk oder Schuhwerkteil nach einem der Ansprüche 1 bis 7, dadurch gekennzeichnet, daß zur Korrektur des pes adduktus (Sichelfuß) und des zugehörigen pes supinatus die Drehvorrichtung den Vor- oder Mittelfußbereich sowohl medial absenkt, als auch zugleich ihn nach lateral drückt.

9. Schuhwerk oder Schuhwerkteil nach einem der Ansprüche 1, 2 und 8, dadurch gekennzeichnet, daß als Drehmittel und/oder Bewegungsmittel ein Gestänge dient.

10. Schuhwerk oder Schuhwerkteil nach Anspruch 9, dadurch gekennzeichnet, daß ein zweiarmiger Hebel (20, 21) vorgesehen und um eine vertikal zur Sohle oder Einlage (16) verlaufende Achse (22) an der Sohle oder Einlage angelenkt ist und daß die Schale oder Platte (17-19) unterseitig einen Vorsprung (23) mit einer schräg zu seiner Bewegungsrichtung verlaufenden Betätigungsfläche (23') aufweist, wobei sich der eine kürzere Querhebelarm (21) im Absenkbereich dieser Betätigungsfläche befindet und mit dem entsprechenden Verschwenken des Hebels einen anderen, längeren Längshebelarm (20) nach lateral (25) zwecks Drehens und Bewegens des Vor- oder Mittelfußbereiches verschwenkt, daß eine den Vorfuß umgreifende und durch einen quer angelegten Tunnel der Sohle verlaufende Schlaufe (26) vorgesehen ist, wobei das vordere Ende des längs verlaufenden Hebelarmes (20) an der Schlaufe mit der lateral gerichteten Zugkraft (25) angreift und daß bevorzugt die vordere Belastungsfläche der Sohle medial tiefer liegt als lateral.

11. Schuhwerk oder Schuhwerkteil nach Anspruch 10, dadurch gekennzeichnet, daß der Längshebel ein das Durchbiegen der Sohle oder Einlage ermöglichendes Gelenk aufweist oder in diese Richtung leicht biegbar ist.

12. Schuhwerk oder Schuhwerkteil nach einem der Ansprüche 1 bis 11, dadurch gekennzeichnet, daß als Dreh- und/oder Bewegungsmittel des Vorfußes in der Sohle im Bereich unter dessen Auftrittsfläche ein Schieber (33, 56) gelagert ist, der etwa quer zur Fußlängsachse verläuft und durch Band- oder Seilzug (35, 53) oder einem Gestänge in seiner Längsrichtung verschiebbar ist, sowie in einer Wirkverbindung mit dem Vorfuß steht.

13. Schuhwerk oder Schuhwerkteil nach Anspruch 12, dadurch gekennzeichnet, daß der Schieber sich etwa in der Ebene der Fußauftrittsfläche befindet und eine davon abweichende Winkellage in dem Sinne einnehmen kann, daß er medial tiefer liegt als lateral, wobei der Schieber bzw. seine Führung lateral etwa am Sohlenrand angelenkt sind und medial darunter ein Raum zur Schwenkung des Schiebers und seiner Führung besteht.

14. Schuhwerk oder Schuhwerkteil nach Anspruch 12 oder 13, dadurch gekennzeichnet, daß an einem der Schieberenden ein von ihm etwa senkrecht nach oben ragender Fortsatz (37, 62) für den zu bewegenden Fußteil und/oder für das Anbringen einer den Fußteil umschließenden Schlaufe (38, 63) vorgesehen ist.

15. Schuhwerk oder Schuhwerkteil nach einem der Ansprüche 12 bis 14, dadurch gekennzeichnet, daß der von der Ferse vermittelte Kraftzug durch Band oder Seil (35) mittels einer Umlenkrolle oder -fläche zur Unterseite des Schiebers (33) geführt ist und dort angreift (36) und daß unterhalb des lateral an der Sohle angelenkten Schiebers (33) zumindest eine Umlenkrolle (40) oder -fläche in verschiedenen Lagen in Längsrichtung des Schiebers an der Sohle derart befestigbar ist, daß mit einem Zug an diesem Band oder Seil der Schieber entweder nur medial abgesenkt, oder nur lateral verschoben oder aber zugleich medial abgesenkt und lateral verschoben wird.

16. Schuhwerk oder Schuhwerkteil nach einem der Ansprüche 1 bis 15, dadurch gekennzeichnet, daß ein Schieber (56) an oder nahe seinem medialen Ende mit dem Fortsatz (62) versehen ist, der direkt oder über eine Schlaufe (63) an der Großzehe angreift, und daß eine Anordnung der Betätigung (Gestänge oder Band bzw. Seil) des Schiebers derart vorgesehen ist, daß mit dem Absenken der Ferse er die Großzehe nach medial verlagert.

17. Schuhwerk oder Schuhwerkteil nach Anspruch 16, dadurch gekennzeichnet, daß ferner eine Seilzugbetätigung (64, 65) für einen vertikalen Druck (Hammerzehenkorrektur) auf die Zehen zwei bis fünf vorgesehen ist, wobei bei Absenken des Rückfußes diese Seilzugbetätigung zusammen mit der Betätigung des Schiebers (56) für die Hallux-valgus-Korrektur der Großzehe erfolgt.

18. Schuhwerk oder Schuhwerkteil nach Anspruch 17, dadurch gekennzeichnet, daß je ein Band- oder Seilzug (64, 65) an den Enden einer Druckplatte (66) angreift, die sich oberhalb der Zehen zwei bis fünf befindet und mit dem Betätigen dieser Seilzüge die vorgenannten Zehen nach unten drückt.

## Claims

1. A shoe orthosis or component of a shoe orthosis with a mechanism for conservative treatment of foot deformities, wherein the pressure of the posterior part of the foot on the shoe orthosis device or component is transmitted to the forefoot region in an intermittently acting force, characterized in that means are provided which are aubjected to said force and which rotate the forefoot around the longitudinal axis of the foot and/or move it substantially transversely to said longitudinal axis about an axis extending perpendicularly to the sole to the outer or inner side, whereby said means act on the inner side of the forefoot to lower it, thus rotating said forefoot.

2. Shoe orthosis or component according to claim 1, characterized in that the rotating means are operatively connected to an arrangement which, in response to the pressure of the posterior part of the foot on the device, produces correction of « bent foot » (talipes valgus) and « flatfoot » (fallen arches and pes supinatus, concurrent with talipes valgus) whereby the rotating means and said correcting arrangement act in opposite rotational directions, i. e., they produce a lowering of the outer side part of the heel and a lowering of the inner part of the metatarsal or forefoot region, wherewith preferably at least one upwardly and downwardly movable stepping shell or plate for support of the foot is provided which, when its heel region is lowered, produces a rotational movement of the forefoot or metatarsal region, and/or transverse movement of the forefoot or metatarsal region, by means of force transmission.

3. Shoe orthosis or component according to claim 1 or 2, characterized in that one or more straps, belts, cords, or cables (7 and 56) serve as the rotating means.

4. Shoe orthosis or component according to claim 3, characterized in that a stepping plate (4″) is provided which is pivotally connected (15) to the sole (1) at the inner border and is present in the posterior, heel region, wherewith said plate bears actuating means (13) underneath it, beneath which means the strap or cord (or straps or cords) (7) pass by, and space is provided under said plate for it to swing downward ; and in that said strap(s) or cord(s) (7) run to the metatarsal or forefoot region and are operatively connected to the rotational means in the respective actuation directions of said rotational means, wherein said strap(s) or cord(s) are actuated (tensioned) according to the block and tackle pulley principle whereby a vertically downward motion of the stepping plate (or similar member) over a certain distance in the region of the heel is converted into an integral-multiple pulling movement of the strap(s) or cord(s), wherein preferably multiple rollers or sliding-surfaces (13) are mounted on the stepping plate and multiple rollers or sliding surfaces (12) are mounted on the sole.

5. Shoe orthosis or component according to claim 4, characterized in that in a « bent foot » and « flatfoot » insole the pulley arrangement operating by the block and tackle principle is disposed in the outer posterior heel region of said insole underneath the stepping plate (4″) which is pivotally connected to the sole (15) at the inner side ; and in that a strap or cord exerts an approximately vertically downward force on the underside of an anterior stepping plate (4′) which is provided underneath the foot in the region of the metatarsus or the forefoot, or else said plate (4′) is pivotally mounted (5) on the outer side of the sole in this region so that its inner part is swingable around the pivot, and said plate (4′) is continued by a forefoot strap on the inner side, and a space is provided under the stepping plate (4′) to permit it to swing downwardly around said pivot (5).

6. Shoe orthosis or shoe orthosis part according to claims 3 to 5, characterized in that the effective length of the strap(s) or cord(s) and the position of said strap(s) or cord(s) is (are) adjustable and is (are) fixable in each adjustment position, in order to adjust the tension for the individual user.

7. Shoe orthosis or shoe orthosis part according to claim 3 or 6, characterized in that a chamber made of a flexible material and containing a liquid is provided under the posterior stepping plate (4″), which chamber may be, e. g., lens-shaped, said strap or cord extending around said chamber so that a downward motion of the stepping plate (4″) causes an expansion of the perimeter of the liquid-containing chamber, thus producing a pulling movement or the strap or cord.

8. Shoe orthosis or shoe orthosis part according to one of claims 1 to 7, characterized in that, for correcting pes adductus (« sickle foot ») and concurrent pes supinatus, the rotational device lowers the inner side part of the forefoot or metatarsal region and at the same time pushes it transversely toward the outer side.

9. Shoe orthosis of part thereof, according to one of the claims 1, 2 and 8, characterized in that the rotational means and/or the transverse motional means are a lever system.

10. Shoe orthosis or part thereof according to claim 9, characterized in that a two-armed lever (20 and 21) is provided which is pivotally mounted to the sole or insole so as to swing around an axis (22) which is vertical to the sole or insole (16), further in that the shell or plate (17 to 19) has a projection (23) on its underside, and an actuating surface (23′) on said projection which surface (23′) extends at an angle to its direction of motion, whereby the shorter, transverse lever arm (21) is disposed in the downward path of travel of said actuating surface, and when said arm (21) is moved about said pivot another, longer, longitudinal lever arm (20) is swung in the transverse direction (25) toward the outer side, so as to rotate and move the forefoot or metatarsal region of the foot ; further in that a loop (26) is provided which extends around and holds the forefoot and extends through a transverse hollow opening in said hole, the anterior end of the longitudinally extending lever arm (20) engaging said loop so that its transversely outwardly directed (25) pulling force acts on said loop ; and in that preferably the anterior inner side load surface of the sole lies lower than the anterior outer side load surface of the sole.

11. Shoe orthosis or part thereof according to claim 10, characterized in that the longitudinal lever arm has a joint which enables the flexing of the sole or insole, or is easily bendable in the vertical direction.

12. Shoe orthosis or part thereof according to claims 1 to 11, characterized in that a sliding member (33 and 56) which acts as a rotational and/or transverse motional means for the forefoot is housed in the sole in the region beneath the stepping surface of said orthosis, said sliding member runs transversely to the longitudinal axis of the foot, is slidable in its own longitudinal direction by means of a pulling strap or cord (35 and 53) or a rod, and is operatively connected to the forefoot.

13. Shoe orthosis or part thereof according to claim 12, characterized in that said sliding member is disposed approximately in the plane of the stepping surface but is movable to a different angular position therefrom in which position said member is lower on its inner side than on its outer side, with said member and/or its guide being pivotally connected substantially at the outer side edge of the sole (on the outer side of said member or guide), and wherewith there is a space situated underneath said sliding member into which said member and its guide may be swung.

14. Shoe orthosis or part thereof according to claim 12 or 13, characterized in that on one of the ends of said sliding member an extension (37 and 62) is provided which extends approximately vertically upward from said member, to accommodate the part of the foot which is to be moved and/or to accommodate the attachment of a loop (38 and 63) for surrounding said foot part.

15. Shoe orthosis or part thereof according to one of claims 12 to 14, characterized in that the tension force supplied from the heel is transmitted to the underside of the sliding member (33) via a strap or cord (35) with the aid of a deflecting roller or deflecting surface, and is applied to said member there (36) ; and in that at least one deflecting roller (40) or deflecting surface is attachable to the sole at a location which is underneath the sliding member (33) (which member is pivotally mounted to the sole on the outer side thereof) and said deflecting roller or rollers may be disposed at various positions along the longitudinal direction of said member, such that when said strap or cord is pulled the inner side of said sliding member is lowered or said member is slid laterally toward the outer side, or the inner side of said member is lowered and at the same time said member is slid laterally toward the outer edge.

16. Shoe orthosis according to one of claims 1 to 15, characterized in that a sliding member (56) is provided at or near the inner side of said member (56) with an extension (62) which engages the great toe directly or via a loop (63) ; and in that a mechanism (including, e. g., a rod, strap, or cord) for moving said sliding member is provided, which moves the great toe in the inward direction when the heel is lowered.

17. Shoe orthosis or part thereof according to claim 16, characterized in that a pulling-cord means (64, 65) is provided, to produce a vertical downward pressure (to correct « hammer toe ») on toes two through five, and when the hindfoot is lowered this pulling-cord means acts together with the motion of the sliding member (56) to correct hallux valgus of the great toe.

18. Shoe orthosis or part thereof according to claim 17, characterized in that each of the pulling-strap or pulling-cord means (64 and 65) acts on an end of a pressure plate (66) disposed above toes two through five, whereby when said strap or cord means is tensioned said toes are pressed downward.

**Revendications**

1. Chaussure orthopédique ou partie de cette chaussure, comportant un dispositif pour le traitement conservatif de malformations du pied, la pression de l'arrière du pied sur la chaussure ou partie de chaussure se transformant en un effort intermittent sur la région antérieure du pied, caractérisée en ce qu'il est prévu des moyens soumis à l'action de la force qui font tourner la région de l'avant du pied autour de l'axe longitudinal du pied et/ou la déplacent de côté (latéralement ou médialement) autour d'un axe dirigé perpendiculairement à la semelle, et en ce que les moyens de rotation de la région de l'avant du pied s'appliquent au côté médial de l'avant du pied, dans le sens de son abaissement.

2. Chaussure ou partie de chaussure selon la revendication 1, caractérisée en ce que les moyens de rotation sont en liaison sous l'action d'une force avec une disposition assurant, lors de la charge du talon, une correction du pied plat valgus, les moyens de rotation et la disposition de correction du pied plat valgus étant dirigés en sens opposé dans leur sens de rotation respectif, c'est-à-dire qu'un abaissement de la partie latérale du talon provoque un abaissement de la région médiale du milieu ou de l'avant du pied, au moins une cuvette ou plaque pouvant se mouvoir vers le haut et vers le bas étant prévue pour l'application du pied et assurant par transmission de force, par l'abaissement de sa région de talon, la rotation et/ou le mouvement de la région de l'avant ou du milieu du pied.

3. Chaussure ou partie de chaussure selon l'une des revendications 1 ou 2, caractérisée en ce que comme moyens de rotation, on utilise un câble ou lien (7, 56) ou des câbles ou liens.

4. Chaussure ou partie de chaussure selon la revendication 3, caractérisée en ce qu'il est prévu une plaque de marche (4″) articulée (15) à la semelle (1) du côté médian et se trouvant en arrière dans la région du talon et qui présente, par-dessous, des moyens d'actionnement (13) sous lesquels passent le ou les câbles ou liens (7), un espace étant prévu en dessous de cette plaque de marche pour son pivotement vers le bas, et en ce que le lien ou câble (ou les liens ou câbles) (7) courent vers la région du milieu ou de l'avant du pied et s'appliquent au moyen de rotation dans le sens d'actionnement momentané, l'actionnement du lien ou câble ou des liens ou câbles s'effectuant selon le principe du palan, de telle sorte qu'un mouvement vertical vers le bas, d'une longueur déterminée, de la plaque de marche, ou similaire, dans la région du talon est transformé en un mouvement de traction du lien ou câble ou des liens ou câbles, qui est un multiple entier de ce mouvement, plusieurs galets ou surfaces de glissement (13) étant, de préférence, disposés sur la surface ou plaque de marche et plusieurs galets ou surfaces de glissement (12) sur la semelle.

5. Chaussure ou partie de chaussure selon la revendication 4, caractérisée en ce que dans le cas d'une doublure pour pied plat valgus, la disposition conçue selon le principe du palan se trouve dans la région latérale postérieure du talon, en dessous de la plaque de marche (4″) qui est articulée médialement à la semelle (15), et en ce qu'un lien ou câble s'applique à une plaque de marche (4′) prévue dans la région du milieu ou de l'avant du pied, à sa face inférieure, avec un sens de traction dirigé verticalement vers le bas, ou y est articulé médialement et se continue par la courroie médiale de l'avant du pied, la plaque de marche (4′) étant articulée latéralement (5) à la semelle et un espace étant prévu sous la plaque de marche pour son pivotement autour de l'articulation (5).

6. Chaussure ou partie de chaussure selon l'une des revendications 3 à 5, caractérisée en ce que la longueur effective du lien ou câble ou des liens ou câbles est réglable en vue d'obtenir une tension individuelle et ils peuvent être fixés dans la position de réglage momentanée.

7. Chaussure ou partie de chaussure selon l'une des revendications 3 à 6, caractérisée en ce qu'en dessous de la plaque postérieure de marche (4″) est prévue une disposition hydraulique de chambre en forme de lentille, formée de matière souple, à contenu liquide, et qu'elle est entourée par le lien ou câble de telle sorte qu'un mouvement vers le bas de la plaque de marche assure une dilatation de la chambre munie du contenu liquide, à sa ligne circonférentielle, et donc, un mouvement de traction sur le câble ou lien.

8. Chaussure ou partie de chaussure selon l'une des revendications 1 à 7, caractérisée en ce que pour la correction du pes adductus (pied falciforme) et du pes supinatus correspondant, le dispositif de rotation abaisse médialement la région de l'avant ou du milieu du pied et en même temps la pousse latéralement.

9. Chaussure ou partie de chaussure selon l'une des revendications 1, 2 et 8, caractérisée en ce que comme moyen de rotation et/ou moyen de mouvement, une tringlerie est utilisée.

10. Chaussure ou partie de chaussure selon la revendication 9, caractérisée en ce qu'il est prévu un levier à deux bras (20, 21) articulé à la semelle ou à la doublure (16) autour d'un axe (22) dirigé verticalement par rapport à la semelle ou à la doublure (16), et en ce que la cuvette ou plaque (17 à 19) présente dans le bas une saillie (23) munie d'une surface d'actionnement (23′) dirigée obliquement par rapport à sa direction de mouvement, l'un des bras de levier transversal (21), plus court, se trouvant dans la région d'abaissement de cette surface d'actionnement et faisant pivoter, par le pivotement correspondant du levier, un bras de levier longitudinal (20), plus long, vers le côté latéral (25), afin de faire tourner et de mouvoir la région de l'avant ou du milieu du pied, en ce qu'il est prévu une boucle (26) entourant l'avant du pied et passant par un tunnel formé transversalement sur la semelle, l'extrémité antérieure du bras de levier dirigé longitudinalement (20) s'appliquant à la boucle avec la force de traction (25) dirigée latéralement, et en ce que, de préférence, la surface antérieure de charge de la semelle est placée plus bas médialement que latéralement.

11. Chaussure ou partie de chaussure selon la revendication 10, caractérisée en ce que le levier longitudinal présente une articulation permettant la flexion de la semelle ou de la doublure, ou est légèrement flexible dans cette direction.

12. Chaussure ou partie de chaussure selon l'une des revendications 1 à 11, caractérisée en ce que comme moyen de rotation et/ou de mouvement de l'avant du pied, dans la semelle est monté, dans la région située sous sa surface de marche, un coulisseau (33, 56) qui est à peu près dirigé transversalement à l'axe longitudinal du pied et qui peut coulisser dans sa direction longitudinale sous l'action d'une traction à lien ou à câble (35, 56) ou d'une tringlerie, et qui est en liaison fonctionnelle avec l'avant du pied.

13. Chaussure ou partie de chaussure selon la revendication 12, caractérisée en ce que le coulisseau se trouve à peu près dans le plan de la surface de marche et peut occuper une position angulaire différente, en ce sens qu'il est placé plus bas médialement que latéralement, le coulisseau ou son guide étant articulés latéralement, par exemple au bord de la semelle, et un espace étant prévu en dessous, médialement, pour le pivotement du coulisseau et de son guide.

14. Chaussure ou partie de chaussure selon l'une des revendications 12 et 13, caractérisée en ce qu'à l'une des extrémités du coulisseau est prévu un prolongement (37, 62) se dressant verticalement de celui-ci vers le haut, pour la partie du pied à mouvoir et/ou pour la disposition d'une boucle (38, 63) entourant la partie de pied.

15. Chaussure ou partie de chaussure selon l'une des revendications 12 à 14, caractérisée en ce que la force de traction communiquée par le talon est conduite par un lien ou câble (35), au moyen d'une poulie ou surface de renvoi, au côté inférieur du coulisseau (33) et s'y applique (36), et en ce qu'en dessous du coulisseau (33) articulé latéralement à la semelle, au moins une poulie (40) ou surface de renvoi peut se fixer à la semelle en différentes positions dans la direction longitudinale du coulisseau, de telle sorte que par une traction sur ce lien ou câble, ou bien le coulisseau s'abaisse seulement médialement, ou bien il coulisse latéralement, ou encore, il s'abaisse médialement et coulisse en même temps latéralement.

16. Chaussure ou partie de chaussure selon l'une des revendications 1 à 15, caractérisée en ce qu'un coulisseau (56) est muni, à son extrémité médiale ou près de celle-ci, du prolongement (62), qui s'applique au gros orteil directement ou par l'intermédiaire d'une boucle (63), et en ce qu'une disposition d'actionnement (tringlerie ou lien ou câble) du coulisseau est prévue de telle sorte que lorsque le talon s'abaisse, le coulisseau déplace le gros orteil vers le côté médial.

17. Chaussure ou partie de chaussure selon la revendication 16, caractérisée en ce qu'il est prévu, en outre, un actionnement par câble de traction (64, 65) pour une poussée verticale (correction des orteils en marteau) sur les 2ème et 5ème orteils, cet actionnement par câble de traction s'effectuant, lors de l'abaissement de l'arrière du pied, en même temps que l'actionnement du coulisseau (56) pour la correction d'hallux valgus du gros orteil.

18. Chaussure ou partie de chaussure selon la revendication 17, caractérisée en ce qu'une traction à lien ou à câble (64, 65) s'applique à chacune des extrémités d'une plaque de pression (66) qui se trouve au-dessus des 2ème à 5ème orteils et qui, lorsque ces tractions à câble sont actionnées, pousse vers le bas les orteils susdits.

Fig. 1

Fig. 2

_Fig. 3_

_Fig. 4_

Fig. 5

Fig.6

0 040 782

*Fig. 7*

Fig. 8

*Fig. 9*

50

51

52

68

49

Fig . 10

XII

Fig. 11

Fig. 12

11

*Fig . 13*